(19) Europäisches Patentamt
European Patent Office
Office européen des brevets

(11) **EP 4 477 727 A1**

(12) **EUROPEAN PATENT APPLICATION**

(43) Date of publication:
**18.12.2024 Bulletin 2024/51**

(51) International Patent Classification (IPC):
**C10G 1/00** (2006.01)     **C10G 1/02** (2006.01)
**C10G 3/00** (2006.01)     **C07C 37/54** (2006.01)
**C08L 97/00** (2006.01)

(21) Application number: **24180447.5**

(22) Date of filing: **06.06.2024**

(52) Cooperative Patent Classification (CPC):
**C10G 1/008; C07C 37/54; C08L 97/005;**
**C10G 1/002; C10G 1/02; C10G 3/40; C10G 3/42;**
**C10G 3/50;** C10G 2300/1014; C10G 2300/4081;
C10G 2300/44

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **13.06.2023  FI 20235662**

(71) Applicant: **UPM-Kymmene Corporation**
**00100 Helsinki (FI)**

(72) Inventors:
• **GUTIERREZ, Andrea**
**00100 Helsinki (FI)**

• **JOKELA, Pekka**
**00100 Helsinki (FI)**
• **KERSTEN, Sascha**
**7512 EE Enschede (NL)**
• **HEESINK, Bert**
**7512 EE Enschede (NL)**
• **POLLEMAN, Lars**
**7512 EE Enschede (NL)**
• **WESTERHOF, Roel**
**7512 EE Enschede (NL)**

(74) Representative: **Boco IP Oy Ab**
**Kansakoulukatu 3**
**00100 Helsinki (FI)**

(54) **SOLVOLYSIS PROCESS**

(57)    The present disclosure relates to the thermal liquefaction of lignin, and more particularly to lignin solvolysis of a lignin feedstock (10) or a lignin-based feedstock (10). The process comprises subjecting a feed mixture (30) of lignin feedstock (10) or lignin-based feedstock (10) and solvent (20) to a thermal liquefaction step by heating (110) the feed mixture (30) at a temperature between 300 and 420 °C and at a pressure below 35 bar, wherein the solvent (20) of the feed mixture (30) is a recycled product fraction (61) separated (120) from the obtained liquid product mix (50). The present disclosure further relates to the use of the obtained product(s).

Figure 1

**Description**

FIELD OF THE DISCLOSURE

The present disclosure relates to the thermal liquefaction of lignin, and more particularly to lignin solvolysis of a lignin feedstock or lignin-based feedstock and a recycled product fraction obtained from the process. More in detail the disclosure relates to a process for converting lignin to renewable liquid product(s), wherein the process comprises heating a feed mixture of lignin feedstock or lignin-based feedstock and recycled product fraction during a thermal liquefaction step at high temperatures and low pressure by recirculating a fraction(s) of the process as solvent. The recycled product fraction comprises at least a fraction which corresponds to a fraction having a boiling point between 300 °C and 600 °C at atmospheric pressure. The present disclosure further relates to the use of the obtained product(s).

BACKGROUND OF THE DISCLOSURE

Biomass is increasingly recognized as a valuable feedstock to be used as a sustainable alternative to petroleum for the production of biofuels and chemicals.
Renewable energy sources represent the potential fuel alternatives to overcome the global energy crises in a sustainable and eco-friendly manner. In future, biofuels and biochemicals may replenish the conventional non-renewable energy resources due to their renewability and several other advantages.
Biofuels and biochemicals are typically manufactured from feedstock originating from renewable sources, including oils and fats obtained from plants, animals, algal materials and fish. One source is lignocellulosic biomass, which refers to plant biomass that is composed of cellulose, hemicellulose, and lignin. Biofuels and biochemicals originating from lignocellulosic biomass can replace fossil fuels from an energy point of view. However, the conversion of cellulose and hemicellulose into fuels and chemicals often leaves lignin as a by-product and lignin removed for example by the kraft process is traditionally burned for its fuel value, providing energy to power the mill.
After cellulose, lignin is the most prevalent biopolymer on earth, but in contrast to other polymers, lignin is more resistant to degradation. Although, utilization of lignin as a renewable polymeric material is recognized, lignin utilization is still limited, especially since high-lignin containing biomasses are susceptible to tar and char formation, and often produce significant amount of solid residues.
Converting biomass into renewable fuels and chemicals usually involves thermal treatment of the biomass and a promising technology is Hydrothermal Liquefaction (HTL). HTL is usually carried out with liquid water at temperatures between 320 °C and 400 °C. To keep the water in the liquid state or supercritical state very high operational pressures of 200 bar or above are needed.
Despite the ongoing research and development in the processing of feedstocks and manufacture of fuels, there is still a need to provide an improved process for converting biomass, particularly lignin, to valuable chemicals and hydrocarbons suitable as fuels or fuel blending components.

BRIEF DESCRIPTION OF THE DISCLOSURE

The invention provides methods for producing renewable liquid product(s) from lignin feedstock obtained from different sources or a mixture of lignin feedstock and one or more other renewable feedstock(s), typically a solid renewable feedstock, preferably sawdust. The method generally involves providing a sole lignin feedstock or a lignin-based feedstock in a feed mixture to be used in a thermal treatment process, preferably a continuous thermal treatment process. A renewable product consisting mainly of oxygen containing hydrocarbons is produced and the product can be used as such, can be upgraded or can be used in the process as a recycled product fraction used as the sole solvent in the feed mixture.
The disclosure is based on the idea of using a lignin feedstock or lignin-based feedstock, together with a certain recycled product fraction produced by the process, as feed mixture for thermal treatment. A process for thermal conversion treatment by solvolysis of the feed mixture, is provided. Due to the chosen recycled product fraction, the solvolysis of the process can be operated at low pressures below 35 bar, and still high yields of good quality liquids are obtained. In an optional pre-treatment step for providing the feed mixture of the process, the lignin feedstock or lignin-based feedstock is mixed with the recycled product fraction used as the sole solvent, whereas in the solvolysis step the feed mixture is liquefied.
An advantage of the process of the disclosure is that the main challenges of prior art hydrothermal liquefaction are avoided, i.e. feeding of the reaction slurry to the reactor at high or moderate pressures and temperatures, operating at high or moderate pressures associated with the presence of water at high temperatures, operating near supercritical temperature and pressure of water and recovering product at high or moderate pressure. Moreover, high temperatures and pressure of water (including salts) under supercritical conditions lead to precipitation of salts that block the equipment.

This can now be avoided. Further, special materials needed due to harsh conditions or higher amount of aqueous phase or wastewater, resulting in high investment costs for HTL plants, can be avoided. It has been noticed that recirculating at least part of a product fraction having a boiling point between 300 °C and 600 °C at atmospheric pressure, for example by using at least part of a fraction having a boiling point between 180 °C and 350 °C at 20 mbar absolute pressure, as at least part of the recycled product fraction, lowers the pressure during solvolysis. Many advantages are directly related to the use of lower pressure, especially relating to equipment construction and operability, safety etc.

Further, by recirculating a recycled product fraction comprising at least part of a fraction(s) having a boiling point between 300 °C and 600 °C at atmospheric pressure as solvent in the solvolysis step, the pressure is kept low, but still the oil yield, i.e. the yield of the liquid product increases or is maintained and formation of coke and undesired polymerization reactions are avoided. Further, the amount of oxygen in the oil products decreases. By adjusting process conditions, the oxygen content of the oil products can be altered. Lower oxygen contents correspond to better product quality.

Further if an optimized lignin feedstock, with a predefined molecular weight is used, this allows the use of lower temperatures in the solvolysis step of the process and thereto heavy fractions after solvolysis can be avoided. Heavy fractions are known to coke at hydrotreatment conditions and thus, the product of a solvolysis using lignin fraction(s) is more suitable to hydrotreatment. Thereto, when heavy fractions of the solvolysis product are minimized, the yield of the hydrotreatable oil (molecules with smaller $M_w$) increases. This oil is suitable as a product as such or for further treatment. Further, solid fractions can be avoided or at least minimized.

If the lignin feedstock, typically a fraction of lignin, is obtained from kraft lignin, the process of the disclosure can alternatively, or in addition, be integrated to a pulp mill. Further, the water from the solvolysis, that is introduced with the feed and/or formed in the process, can optionally be returned to the pulp mill. The formation of aqueous phase in solvolysis indicates that deoxygenation took place during the reaction.

Further integration of a pulp mill with the process for converting fractions of lignin to a renewable product is achieved when lignin is extracted from black liquor, the liquid separated from pulp in kraft pulping. Extracting the lignin from the back liquor also causes off-loading of the recovery boiler of the pulp mill. Thereto fractions unsuitable for solvolysis can be returned to the pulping process. Further, as the separation of kraft lignin takes place before the solvolysis, the cooking chemicals are returned to the pulping process at an earlier stage, making sure that the chemical balance of the pulp mill is maintained.

In order to reduce the consumption of external energy, heat from one or more product streams can be used to heat up feed streams.

The process of the disclosure is especially suitable for converting lignin to valuable chemicals, such as hydrocarbons and/or oxygen containing hydrocarbons suitable as fuels, as fuel blending components or as feedstock for fuels and in chemicals production.

BRIEF DESCRIPTION OF THE DRAWINGS

In the following the disclosure will be described in greater detail by means of preferred embodiments with reference to the accompanying drawings, in which

Figure 1 shows a schematic flow diagram representing one embodiment of the conversion process;

Figure 2 shows a schematic flow diagram representing one embodiment of the conversion process without pre-treatment; and

Figure 3 shows average molecular weight ($M_w$) distribution for fractions obtained by a process according to the disclosure (log g/mol).

DETAILED DESCRIPTION OF THE DISCLOSURE

An industrially effective and sustainable process for recovering renewable products from lignin is provided, with high yield of liquid product(s), and where lignin feedstock is effectively and economically converted to renewable products. The liquid product(s) and especially fractions thereof are particularly suitable as feedstock for manufacturing biofuels and biochemicals by hydroprocessing.

A process is proposed for converting lignin feedstock or lignin-based feedstock to renewable liquid product(s) as well as use of the obtained product(s). The object of the disclosure is achieved by the process and use of the obtained product(s) as characterized by what is stated in the independent claims. The preferred embodiments of the disclosure are disclosed in the dependent claims.

According to an embodiment of the disclosure the optional pre-treatment step of the process comprises preparing a feed mixture by mixing lignin feedstock or lignin-based feedstock with a recycled product fraction produced by the process, which is used as solvent, at a temperature between 80 and 220 °C, at a pressure from 1 to 10 bar, for 5 - 30 min, including the temperature being a temperature between two of the following temperatures; 80 °C, 85 °C, 90 °C, 95 °C, 100 °C, 110 °C, 120 °C, 130 °C, 140 °C, 150 °C, 160 °C, 170 °C, 180 °C, 190 °C, 200 °C, 210 °C and 220 °C for the heating of the mixture at a pressure from 1 to 10 bar, preferably 1 to 5 bar, more preferably 1 to 3 bar. The reaction time, or the residence time in case this step is part of the continuous process, is typically 5-30 minutes, preferably 5-15 minutes, not including the heating time. In the embodiments of the disclosure the pre-treatment step can be carried out batch-wise or in continuously operated reactors. The mixing of the feedstock can be facilitated by mechanical treatment using different kind of mechanical equipment such as stirrer, pump etc, for example a piston pump. The obtained feed mixture is pumpable. During the mixing the lignin feedstock dissolves, at least partly, in the solvent.

In embodiments of the disclosure, a feed mixture is, typically after an optional pre-treatment step, fed to a thermal conversion step, a solvolysis step by thermal liquefaction, where the mixture is heated to a temperature between 300 and 420 °C. At this temperature the pressure typically increases up to between 1 bar (0.1 MPa) and 35 bar (3.5 MPa). The reaction time, or the residence time in case this step is part of the continuous process, i.e. the time the temperature and pressure of the solvolysis step is maintained, is typically 2 - 60 minutes, preferably 5 - 40 minutes, more preferably 5-30 minutes, most preferably 10 - 30 minutes, including the time being between two of the following; 2, 3, 4, 5, 10, 15, 20, 25, 30, 36, 40, 45, 50, 55, 60 minutes. A product mix is obtained, and it is typically directed to a separation step, typically comprising washing and/or filtration, where solids/char is typically separated from the liquid product(s). Typically, if an aqueous phase is formed during the thermal liquefaction, it is separated from the product before the product mix is processed further and/or a fraction of the product mix or the liquid product mix is recirculated. The aqueous phase can be removed for example by decanting or as part of the washing. Water can be formed in the solvolysis due to deoxygenation reactions. Typically, the separated solids form a cake comprising oil. In order to increase the oil yield, this oil is optionally separated from the solids by washing or separating by steam. Optionally the solids are first washed and then separated for example by filtration, and/or sedimentation. The solids, if present and separated, are typically unconverted heavy compounds of lignin or the result of polymerization at the reaction conditions. At least part of said liquid product(s) are recirculated back to the pre-treatment step and/or to be part of the feed mixture as a recycled product fraction used as solvent.

According to embodiments of the disclosure the product mix or part of it, preferably part of the liquid product mix, is directed to fractionation before recirculating part of the liquid product to the pre-treatment step and/or to be part of the feed mixture. Typically, several fraction(s) such as light, middle, and heavy fraction(s) as well as optionally gases and a bottom residue fraction are separated. The fractionation may be for example fractional distillation utilizing at least one fractionation distillation column. Typically, at least part of the fraction having a boiling point between 300 °C and 600 °C at atmospheric pressure, which for example corresponds to a fraction having a boiling point between 180 °C and 350 °C at 20 mbar absolute pressure, preferably a fraction having a boiling point between 180 °C and 350 °C at 20 mbar absolute pressure, is used as the solvent or part of the solvent in the feed mixture of the process and optionally part of this fraction and/or other fraction(s) are upgraded by hydroprocessing. Optionally part of the product mix is directed to hydroprocessing, such as hydrotreatment or hydrocracking instead of or in addition to being directed to fractionation.

In the present specification and claims, the following terms have the meanings defined below.

The term "lignin feedstock" as used herein, refers to lignin obtained from different lignin sources. The lignin feedstock is typically a fraction of lignin chosen based on its average molecular weight. One example is lignin feedstock obtained from kraft lignin, but the lignin could also be obtained from other sources such as lignosulfonate lignin, soda lignin, organosolv lignin or lignin obtained from lignocellulosic ethanol process. More in general lignin is a complex water-insoluble, long-chain heterogeneous organic polymer composed largely of phenylpropane units which are most commonly linked by ether bonds. Oxidative coupling of primarily three p-hydroxycinnamyl alcohols (monolignols): p-coumaryl, coniferyl and sinapyl alcohols results in lignin. Lignin have generally been classified into three major groups based on the chemical structure of their monomer units: softwood lignin, hardwood lignin, and grass lignin. Hardwood lignin consists mainly of guaiacyl and syringyl units and low levels of p-hydroxyphenyl. Conifer lignin has higher levels of guaiacyl units and low levels of p-hydroxyphenyl. Grasses comprise guaiacyl, syringyl and p-hydroxyphenyl units. In some embodiments of the disclosure, the feedstock is typically a fraction of kraft lignin, which is separated from black liquor. The feedstock of kraft lignin essentially consists of lignin, i.e. the feedstock is essentially free of impurities, such as metals or ash and/or residues from black liquor, such as cellulose, hemicellulose, methanol, and cooking chemicals from the kraft process.

The term "lignin-based feedstock", as used herein, refer to a feedstock comprising or consisting of a mixture of lignin feedstock and one or more other renewable feedstock(s), typically a solid renewable feedstock, preferably sawdust. Other solid renewable feedstocks, as used herein, refers to solid forest industry residues, including forest residues. Typically, the solid forest industry residues are, bark, sawdust, wood chips and other wood residues of softwood and hardwood residues from the forest industry as well as cutting residues, for example branches and treetops of softwood and hardwood. The amount of other renewable feedstock, preferably sawdust, is typically at most 70 w-%, preferably at most 60 w-%, more preferably at most 50 w-%. Typically, the amount of other renewable feedstock, preferably sawdust, is between 1 - 70 w-%. Typically, the lignin-based feedstock comprises 30 - 99 w-% lignin feedstock and 1 - 70 w-% other renewable feedstock, preferably solid forest industry residues, more preferably bark, sawdust, wood chips, branches, treetops or mixtures thereof, most preferably sawdust.

The term "solvent", as used herein, refer to the solvent used as part of the feed mixture, optionally for pre-treatment of the lignin feedstock or lignin-based feedstock of the process or used as solvent at start-up of the process, before a recycled product fraction to be recycled in the process is obtained from the process itself. The "recycled product fraction", recirculated from the process itself, comprises one or more fraction(s) separated by fractionation and at least comprises part of a "first fraction" (middle fraction) which corresponds to, is similar or comparable to a fraction having a boiling point between 300 °C and 600 °C at atmospheric pressure, such as a fraction having a boiling point between 180 °C and 350 °C at 20 mbar absolute pressure, preferably the recycled product fraction comprises at least part of a fraction having a boiling point between 180 °C and 350 °C at 20 mbar absolute pressure. The recycled product fraction typically further comprises a "second fraction" (light fraction) which corresponds to, is similar or comparable to a fraction having a boiling point between above 120 °C and below 300 °C at atmospheric pressure, such as fraction(s) having a boiling point between above 100 °C and below 180 °C at 20 mbar absolute pressure, preferably the recycled product fraction comprises at least part of a second fraction having a boiling point between above 100 °C and below 180 °C at 20 mbar absolute pressure. After start-up of the process the solvent, i.e. the recycled product fraction, is entirely recirculated from the process itself and the lignin or lignin-based feedstock is the sole feedstock added to the process.

The term "oil fraction", as used herein, refer to the liquid product or part thereof, obtained from the thermal liquefaction of the process and partly recirculated to the feed mixture of the process.

The term "feed mixture", as used herein, is the homogeneous or heterogenous mixture obtained after mixing lignin feedstock or lignin-based feedstock with solvent according to the disclosure.

The terms "fraction", "first fraction", "second fraction", "product fraction", "middle fraction", "light fraction", "heavy fraction", "volatile fraction", as used herein, refers to fraction(s) obtained from the process itself by fractionation. A person skilled in the art can vary the fractionation or distilling conditions and change the temperature cut point as desired to obtain any desired fraction, boiling in the predetermined ranges. By changing temperature and pressure the skilled person can obtain corresponding fraction(s) to fraction(s) having the claimed boiling point at atmospheric pressure, thus receiving fractions having corresponding features, leading to similar results when the fraction is used as the solvent in the feed mixture of the process of the disclosure. The atmospheric pressure, i.e. standard atmosphere (atm) is a unit of pressure defined as 101.325 kPa The corresponding fractions typically comprises corresponding compounds and/or has a corresponding average molecular weight. Typically, the corresponding fraction(s) is obtained at a pressure chosen from between 2 mbar and 1 bar. Low pressures are preferred to avoid coking as lower temperatures can be used. The fraction, named first fraction in the present disclosure, is a fraction having a boiling point between 300 °C and 600 °C at atmospheric pressure. These fraction(s) are typically named "middle fraction(s)". Typically the first fraction essentially consists of or comprises substantially di-, tri- and tetra-aromatics, preferably at least 90 wt-% di-, tri- and tetra-aromatics, more preferably at least 95 wt-% di-, tri- and tetra-aromatics. Part of the di-, tri- and tetra-aromatics are oxygen containing di-, tri- and tetra-aromatics. Preferably the fraction corresponding to a fraction having a boiling point between 300 °C and 600 °C at atmospheric pressure, is a fraction having a boiling point between 180 °C and 350 °C, at 20 mbar absolute pressure (mbara). Preferably the first fraction, i.e. the middle fraction is a fraction having a boiling point between 180 °C and 350 °C, at 20 mbar absolute pressure (mbara). Typical middle fraction(s) comprises β-aryl ether substructures (β-O-4); B: resinol substructures (β-β); C: phenylcoumaran substructures (β-5), biphenyl substructures (5-5) and/or biphenyl ether (5-O-4). The middle fraction typically has a molecular weight of between 150 g/mol and 500 g/mol. A fraction named "second fraction" in the present disclosure, is a fraction having a boiling point between above 120 °C and below 300 °C at atmospheric pressure. These fraction(s) are typically named "light fraction(s)". Typically, the second fraction essentially consists of or comprises substantially monoaromatics, preferably at least 90 wt-% monoaromatics, more preferably at least 95 wt-% monoaromatics. Part of the monoaromatics are oxygen containing monoaromatics. Preferably, the fraction corresponding to a fraction having a boiling point between above 120 °C and below 300 °C at atmospheric pressure, is a fraction having a boiling point between above 100 °C and below 180 °C, at 20 mbar absolute (mbara) pressure. Preferably the second fraction, i.e. the light fraction is a fraction having a boiling point between above 100 °C and below 180 °C, at 20 mbar absolute pressure (mbara). Typically, light fraction(s) has an average molecular weight of approximately 40 - 200 g/mol (based on polystyrene calibration) and typically comprises 5- and 6-carbon cyclic structures with double bonds or saturated ring, containing keto-groups and/or 1-3-methyl groups, phenolic structures with methyl-, methoxy-, propenyl-, carboxyl- side groups, and hydrated phenantrene 3-ring structures with 3-4 double bonds in the ring and side chains. A fraction having a boiling point above 350 °C, at 20 mbar absolute pressure (mbara) is typically named "heavy fraction". Heavy fraction(s) typically has an average molecular weight of above 450 g/mol. Volatiles are typically removed as an aqueous fraction, i.e. a fraction, comprising water and volatiles, having a boiling point at or below 120 °C at atmospheric pressure. Typically, the first fraction does essentially not consist of compounds having a boiling point below 120 °C at atmospheric pressure. Typically, the second fraction does essentially not consist of compounds having a boiling point below 120 °C at atmospheric pressure.

The kraft lignin feedstock of some embodiments of the disclosure essentially consists of lignin, i.e. the lignin feedstock, typically a fraction of lignin, is essentially free of impurities, such as metals as part of ash and/or residues from black liquor, such as cellulose, hemicellulose, methanol, and cooking chemicals from the kraft process. Typically, the lignin feedstock comprises below 10 wt%, preferably below 5 wt%, more preferably below 1 wt%, most preferably below 0.1 wt% of residues from the black liquor.

In an embodiment of the disclosure a process for converting lignin to renewable liquid product(s) is provided, wherein the process comprises the following steps,

- providing a feed mixture (30) of lignin feedstock (10) and recycled product fraction (20) or of lignin-based feedstock (10), preferably a lignin-based feedstock consisting of lignin feedstock and sawdust, and recycled product fraction (20); optionally by preparing the feed mixture by mixing lignin feedstock or lignin-based feedstock with a solvent consisting of a recycled product fraction produced by the process, at a temperature between 80 and 220 °C, at a pressure from 1 bar to 10 bar, for 5 - 30 min, or optionally by preparing the feed mixture by mixing lignin feedstock with a solvent consisting of a recycled product fraction produced by the process, at a temperature between 80 and 220 °C, under at a pressure from 1 bar to 10 bar, for 5-30 min, followed by filtration and obtaining the feed mixture of lignin fraction(s) and solvent as the filtrate;

- treating said feed mixture in a thermal liquefaction step, preferably a continuous thermal liquefaction step, by heating the feed mixture at a temperature between 300 and 420 °C, and maintaining said temperature for 2 - 60 minutes at a pressure below 35 bar;

- obtaining a product mix;

- obtaining one or more fraction(s) by separating at least a liquid product mix and an aqueous phase from the product mix, and fractionating said liquid product mix to obtain one or more fraction(s); and

- recirculating at least part of a first fraction (middle fraction(s)) having a boiling point between 300 °C and 600 °C at atmospheric pressure, such as at least part of a fraction corresponding to fraction(s) having a boiling point between 180 °C and 350 °C at 20 mbar absolute pressure, preferably at least part of a fraction(s) having a boiling point between 180 °C and 350 °C at 20 mbar absolute pressure as the recycled product fraction. The recycled product fraction typically further comprises at least part of a second fraction (light fraction(s)) corresponding to a fraction having a boiling point between above 120 °C and below 300 °C at atmospheric pressure, such as fraction(s) corresponding to fraction(s) having a boiling point between above 100 °C and below 180 °C at 20 mbar absolute pressure, preferably a second fraction having a boiling point between above 100 °C and below 180 °C at 20 mbar absolute pressure. Typically, the recycled product fraction consists of 60 - 100 wt-% of said first fraction (middle fraction(s)) and 0 - 40 wt-% of said second fraction (light fraction(s)). Typically, the first fraction essentially consists of di-, tri and tetra-aromatics and the second fraction essentially consists of monoaromatics. The recycled product fraction typically essentially consists of at least di-, tri- and tetra-aromatics and optionally thereto of monoaromatics. The recycled product fraction typically essentially consists of compounds with a structure and molecular weight which does not increase the pressure during the thermal liquefaction above 35 bar, while still keeping the feed mixture in liquid phase. The fraction(s) used for the recycled product fraction is separated and/or fractionated from the product mix or the liquid product mix, preferably by fractionation, such as distillation, evaporation, liquid-liquid extraction, ultra filtration and nanofiltration. The aqueous phase typically comprises water, small organic acids, such as acetic acid and/or formic acid and optionally also methanol.

In an embodiment of the disclosure a process for converting lignin to renewable liquid product(s) is provided, wherein the process comprises the following steps,

- providing a feed mixture of lignin feedstock and recycled product fraction or of lignin-based feedstock, preferably a lignin-based feedstock consisting of lignin feedstock and sawdust, and recycled product fraction;

- treating said feed mixture in a thermal liquefaction step, preferably a continuous thermal liquefaction step, by heating the feed mixture at a temperature between 300 and 420 °C, and maintaining said temperature for 2 - 60 minutes at a pressure below 35 bar;

- obtaining a product mix;

wherein the recycled product fraction, used as sole solvent in the feed mixture, is provided by separating a liquid product mix and an aqueous phase from said product mix, fractionating one or more fraction(s) from said liquid product mix, or part thereof, at least a first fraction having a boiling point between 300 °C and 600 °C at atmospheric pressure, and wherein at least part of said first fraction is used as said recycled product fraction or part thereof. The recycled product fraction typically further comprises at least part of a second fraction (light fraction(s)) corresponding to a fraction having a boiling point between above 120 °C and below 300 °C at atmospheric pressure. Typically, the recycled product fraction consists of 60 - 100 wt-% of said first fraction (middle fraction(s)) and 0 - 40 wt-% of said second fraction (light fraction(s)). Typically, the first fraction essentially consists of di-, tri and tetra-aromatics and the second fraction essentially consists of monoaromatics. The recycled product fraction typically essentially consists of at least di-, tri- and tetra-aromatics and optionally thereto of monoaromatics. The recycled product fraction typically essentially consists of compounds with a structure and molecular weight which does not increase the pressure during the thermal liquefaction above 35 bar, while still keeping the feed mixture in liquid phase. The fraction(s) used for the recycled product fraction is separated and/or fractionated from the product mix or the liquid product mix, preferably by fractionation, such as distillation, evaporation, liquid-liquid extraction, ultra filtration and nanofiltration. The aqueous phase typically comprises water, small organic acids, such as acetic acid and/or formic acid and optionally also methanol.

According to embodiments of the disclosure the recycled product fraction comprising at least part of a first fraction having a boiling point between 300 °C and 600 °C at atmospheric pressure and optionally part of a second fraction having a boiling temperature between above 120 °C and below 300 °C at atmospheric pressure, does not comprise a fraction having a boiling point at or below 120 °C at atmospheric pressure (aqueous fraction or volatile fraction) and/or a fraction having a boiling point above 600 °C at 20 mbar absolute pressure (heavy fraction). When the recycled product fraction(s) does not comprise fraction(s) having a boiling point at or below 120 °C at atmospheric pressure (aqueous fraction), high temperatures can be avoided and correspondingly as the recycled product fraction does not comprise fraction(s) having a boiling point above 600 °C at atmospheric pressure (heavy fraction) coking is avoided.

According to embodiments of the disclosure the solvent used for starting up the process, before steady state is reached, is typically a commercial solvent product or hydrocarbon fraction product from another process. By time, this product is removed to be replaced by the recycled product fraction(s) produced by the process. The removal of the solvent used for starting up the process is faster if the boiling point of the product used as solvent is lower than the boiling point of the recirculated fraction(s) used as solvent in steady state.

The amount of lignin feedstock or lignin-based feedstock is typically 5 wt% to 35 wt% and the amount of solvent is typically 65 wt% to 95 wt%, together making up the feed mixture of the embodiments of the disclosure. Preferably the amount of lignin is 8 wt% to 25 wt%, more preferably 10 to 20 wt%, including the amount of lignin being between two of the following amounts; 5 wt%, 6 wt%, 7 wt%, 8 wt%, 9 wt%, 10 wt%, 15 wt%, 20 wt%, 25 wt%, 30 wt% and 35 wt% of the feed mixture and the rest is recycled product fraction.

According to the embodiments of the disclosure, the temperature of the thermal liquefaction step is adjusted to a temperature selected from between 300 °C and 420 °C, preferably between 300 °C and 400 °C, more preferably between 360 °C and 400 °C, including the temperature being a temperature between two of the following temperatures; 300 °C, 310 °C, 320 °C, 330 °C, 340 °C, 350 °C, 360 °C, 370 °C, 380 °C, 390 °C, 400 °C, 410 °C and 420 °C for the heating of the feed mixture, optionally the feed mixture of the pre-treatment step, wherein the pressure remains below or at 35 bar, preferably between 1 and 35 bar, more preferably between 1 bar and 30 bar, more preferably between 1 and 10 bar, including the pressure being between two of the following pressures; 1 bar, 2 bar, 5 bar, 10 bar, 15 bar, 20 bar, 25 bar, 30 bar or 35 bar.

The reaction time of the thermal liquefaction step, or residence time, in case this step is part of a continuous process, is typically 2-60 minutes, preferably 5-40 minutes, most preferably 5-30 minutes not including the heating time.

The process of the embodiments of the disclosure, or parts thereof, can be a continuous, batch or semi-batch process.

In an embodiment of the disclosure, the renewable liquid product of the disclosure comprising oil may be subjected to a further fractionation step. A gaseous fraction, if any, one or more light and/or middle liquid fraction(s), and a heavy liquid fraction may be obtained. Further, a bottom residue fraction, typically comprising large molecules and solids, such as ash, may be separated.

In embodiments of the disclosure, the product mix or the liquid product mix or fractions thereof may be used as a renewable product, for example in the production of chemicals, in marine fuel applications or applications relating to heating or it may be directed to a hydroprocessing step.

In further embodiments of the disclosure, fraction(s) obtained by fractionation of the optionally washed and optionally filtered product mix or liquid product mix, preferably light and/or middle fraction(s), may be directed to a hydroprocessing step to obtain hydroprocessed oil, which can be used inter alia in production of chemicals, marine fuel and/or drop-in fuels such as diesel, naphtha, and jet-fuel.

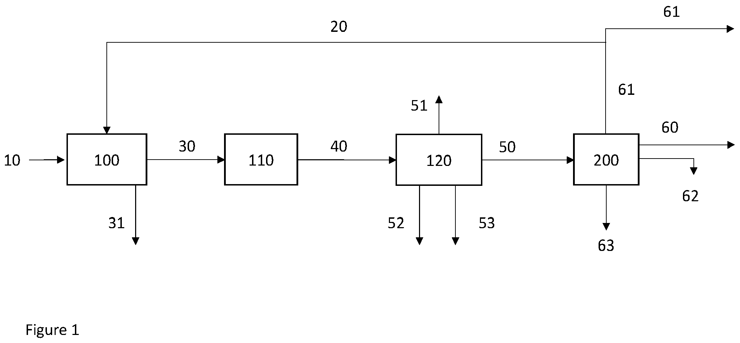

In Figure 1 lignin feedstock 10 or lignin-based feedstock 10 is fed to an optional pre-treatment step 100 together with a recycled product fraction as solvent 20. The mixture is filtrated (not shown) and a feed mixture 30 of lignin fraction (not shown) and recycled product fraction is obtained and solids 31 are removed. The feed mixture 30 is fed to a thermal liquefaction step 110 where it is heated. The product mix 40 obtained from the thermal liquefaction step 110 is directed to a separation step 120, typically comprising washing and filtration where an aqueous phase 53 and a liquid product mix 50 are separated, optionally also gas 51 char/solids 52 are separated. The liquid product mix 50 is directed to fractionation 200 and/or optionally part of the liquid product mix 50 is directed to hydroprocessing, such as hydrotreatment or hydrocracking (not shown). The fractionation step 200 may for example be fractional distillation utilizing at least one fractionation distillation column. An optional gaseous fraction (not shown), a light fraction 60, a middle fraction 61, a heavy fraction 62 and an optional bottom residue fraction 63 are separated. At least part of the middle fraction 61 and optionally part of the light fraction 60 (not shown) is recirculated to be used as solvent 20 in the pre-treatment step 100. Optionally the light fraction 60 or part of it and/or part of the middle fraction 61, is directed to hydroprocessing and/or the heavy fraction 62 is directed to a recovery boiler (not shown in the figure).

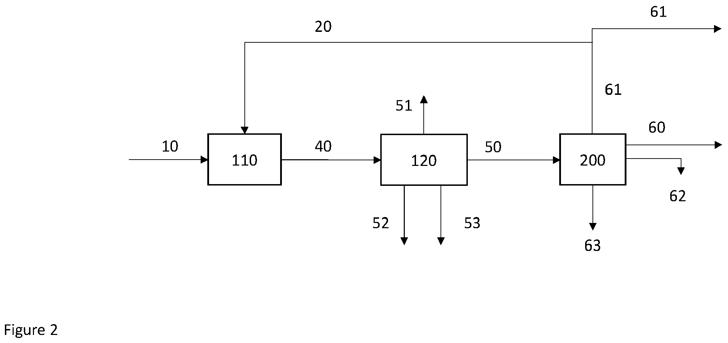

In Figure 2 lignin feedstock 10 or lignin-based feedstock 10 and a recycled product fraction as solvent 20 is fed to a thermal liquefaction step 110 where they are heated. The product mix 40 obtained from the thermal liquefaction step 110 is directed to separation step 120, typically comprising washing and filtration where an aqueous phase 53 and a liquid product mix 50 are separated, optionally also gas 51 and/or char/solids 52 are separated. The liquid product mix 50 is directed to fractionation 200 and/or optionally part of the liquid product mix 50 is directed to hydroprocessing, such as hydrotreatment or hydrocracking (not shown). The fractionation step 200 may for example be fractional distillation utilizing at least one fractionation distillation column. An optional gaseous fraction (not shown), a light fraction 60, a middle fraction 61, a heavy fraction 62 and an optional bottom residue fraction 63 are separated. At least part of the middle fraction 61 and optionally part of the light fraction 60 (not shown) is recirculated to be used as solvent 20 in the pre-treatment step 100. Optionally the light fraction 60 or part of it and/or part of the middle fraction 61, is directed to hydroprocessing and/or the heavy fraction 62 is directed to a recovery boiler (not shown in the figure).

**Lignin**

The lignin feedstock of the embodiments of the disclosure is obtained from different sources and produced in different ways that involves separation of lignin. Examples of different lignin sources are kraft lignin obtained from kraft pulping, lignosulfonate lignin obtained from a sulfite pulping process, soda lignin from pulp mills using a soda pulping process and organosolv lignin where lignin is isolated from carbohydrates in lignocellulosic biomass using organic solvents, also the lignin obtained in the lignocellulosic ethanol production.

Typically, the lignin feedstock is kraft lignin, which essentially consists of lignin, i.e. the feedstock is essentially free of impurities, such as metals or ash and/or residues from black liquor, such as cellulose, hemicellulose, methanol, sulphur compounds and cooking chemicals from the kraft process, preferably the lignin feedstock comprises less than 10 wt%, preferably the lignin comprises less than 5 wt%, more preferably less than 1 wt%, most preferably less than 0.1 wt% of residues from the black liquor. Preferably the lignin is cellulose-free.

The kraft process (also known as kraft pulping or sulfate process) is a process for conversion of wood into wood pulp, which consists of almost pure cellulose fibers, the main component of paper. The process involves the conversion of wood to pulp using an aqueous mixture containing sodium hydroxide and sodium sulphide. These chemicals remove the lignin links between cellulose fibres, thereby releasing the fibres and producing water-soluble compounds. The kraft pulping process is well-known and it is known that it removes most of the lignin originally present in the wood. The liquid separated from pulp is commonly referred to as "crude black liquor" or "weak black liquor". The term "black liquor" refers to the aqueous liquid residue of the kraft pulping process which has been separated from solid wood pulp (cellulose fibres). The black liquor contains dissolved wood degradation products such as lignin and hemicellulose fragments, as well as methanol, sulphur compounds and dissolved inorganic solids such as spent pulping chemicals. The methanol content is typically in the range of 0.5-1.5 wt.% based on the mass of dry solids. Sulphur compounds are typically contained in the black liquor in an amount of 2-7 wt.% based on the mass of dry solids.

An acidification process has been considered as an efficient and economical process for the isolation of lignin from black liquor. One example of black liquor acidification for lignin extraction is adding acid e.g., sulfuric acid to change the pH of black liquor. Other typical processes for obtaining lignin from black liquor are using filtration and ultrafiltration.

A typical lignin feedstock of the embodiments of the disclosure is a composition comprising 15-98 wt% organics, 0.1 - 15 wt% ash and/or 0 - 45 wt% moisture. Typically, the oxygen content of the composition is below 40 wt%, preferably from 20 to 36 wt%, most preferably from 25 - 36 wt% daf (dry-ash-free, i.e. without taking into account possible ash and moisture content). In preferred embodiments of the disclosure; the amount of organics is preferably 30 - 98 wt%, more preferably 30 - 70 wt%, most preferably 50 - 70 wt%; the amount of ash is preferably 0.1 - 7 wt%, more preferably 0.1 - 3 wt%, most preferably 0.1 - 1 wt%, including the ash content being between two of the following values; 0.1 wt%, 0.2 wt%, 0.3 wt%, 0.4 wt%, 0.5 wt%, 1 wt%, 1.5 wt%, 2 wt%, 2.5 wt%, 3 wt%, 3.5 wt%, 4 wt%, 5 wt%, 6 wt%, 7 wt%, 8 wt%, 9 wt%, 10 wt%, 11 wt%, 12 wt%, 13 wt%, 14 wt% and 15 wt%; and/or the amount of moisture is preferably 0 - 35 wt%, more preferably 0 - 20 wt%, most preferably 0 - 5 wt% including the amount of moisture being between two of the following values; 0 wt%, 0.1 wt%, 0.2 wt%, 0.3 wt%, 0.4 wt%, 0.5 wt%, 1 wt%, 2 wt%, 5 wt%, 10 wt%, 15 wt%, 20 wt%, 25 wt%, 30 wt%, 35 wt%, 40 wt% and 45 wt%,. If needed, the lignin feedstock can be dried.

In some embodiments of the disclosure the lignin feedstock can be chosen based on its molecular weight and optionally also its polydispersity. Typically, the lignin feedstock is fraction(s) of lignin, obtained by filtration, nanofiltration, acid precipitation process or membrane fractionation. Lignin can be subjected to gradient acid precipitation to obtain lignin fractions with narrow molecular weight distribution. During gradient acid precipitation lignin fractions obtained at higher pH value exhibit higher molecular weight compared with the fractions obtained at lower pH. The fractionation can also be achieved by dissolving lignin in solvent used in the solvolysis, followed by filtration where after a feed mixture of a lignin fraction and solvent is obtained as the filtrate. Since small particles have larger surface area they dissolve better and a fraction of lignin with a lower molecular weight is obtained as filtrate.

In some embodiments of the disclosure, the average molecular weight of the lignin feedstock is below 6500 g/mol, preferably below 4000 g/mol, more preferably below 2000 g/mol, most preferably below 1500 g/mol. Typically, the polydispersity of the lignin feedstock is 1.2 to 6, preferably 1.2 to 4.5, more preferably 1.2 to 2.5.

In some embodiments of the disclosure the lignin feedstock is typically a fraction of kraft lignin, which is separated from black liquor, having an average molecular weight below 6500g/mol, preferably below 4000 g/mol, more preferably below 2000 g/mol, most preferably below 1500 g/mol.

The molecular weight distribution can for example be determined using Gel Permeation Chromatography (GPC). GPC is an analytical technique that separates dissolved macromolecules by size based on their elution from columns filled with a porous gel. GPC/SEC employs a stagnant liquid present in the pores of beads as the stationary phase, and a flowing liquid as the mobile phase. The mobile phase can therefore flow between the beads and also in and out of the pores in the beads. The separation mechanism is based on the size of the polymer molecules in solution. Bigger molecules will elute first. Small molecules that can enter many pores in the beads take a long time to pass through the column and therefore exit the column slowly. To determine the molecular weights of the components of a polymer sample, a calibration with standard polymers of known weight must be performed. Values from the unknown sample are then compared with the calibration graph. This method is relative and retention times depends on the used column material, eluent and how similar the used standards are compared to the samples.

**Forest industry residues**

The forest industry residues of the embodiments of the disclosure are typically residues from forest industry such as bark, sawdust, wood chips, other wood residues or a combination thereof. The forest industry residues are typically produced at an industry area, such as sawdust by timber industry or de-barking at pulp industry.

In some embodiments of the disclosure, the solid forest industry residues are solid forest residues such as cutting residues, for example branches and treetops.

Typically, the solid second renewable feedstock is solid forest industry residues, including solid forest residues, preferably the solid second renewable feedstock is bark, sawdust, wood chips, branches, treetops or mixtures thereof, more preferably bark, sawdust, wood chips or mixtures thereof.

Typical compositions of sawdust, bark and forest residues of the embodiments of the disclosure are shown in Table 1.

Table 1. Composition of different forest industry residues by weight.

| Description | Unit | Method | Spruce sawdust | Pine sawdust | Spruce bark | Pine bark | Forest residue |
|---|---|---|---|---|---|---|---|
| Dry content 105°C | % | Internal | 46.2 | 45.9 | 55.8 | 42.2 | 42 |
| Carbon, C | % | ASTM D5291 | 49.40 | 49.93 | 49.50 | 54.10 | 47.2 |
| Hydrogen, H | % | ASTM D5291 | 6.34 | 6.36 | 5.93 | 5.57 | 5.4 |
| Nitrogen, N | % | ASTM D5291 | 0.10 | 0.08 | 0.27 | 0.23 | 0,7 |
| Sulphur, S | % | ASTM D4239 | 0.00 | 0.00 | 0.02 | 0.02 | 0.005 |

(continued)

| Description | Unit | Method | Spruce sawdust | Pine sawdust | Spruce bark | Pine bark | Forest residue |
|---|---|---|---|---|---|---|---|
| Oxygen, O | % | ASTM D5291 | 43.31 | 43.01 | 41.90 | 38.00 | 46,65 |
| Ash 550°C | % | SFS-EN 14775 | 0.3 | 0.2 | 2.4 | 2.1 | 3.5 |

Typically, the ash content of the solid forest industry residue is 0.1 - 5 wt%, preferably 0.1 - 4 wt%, more preferably 0.1 - 2.5, most preferably 0.1 - 1 wt%, including the ash content being between two of the following values; 0.1 wt%, 0.2 wt%, 0.3 wt%, 0.4 wt%, 0.5 wt%, 1 wt%, 1.5 wt%, 2 wt%, 2.5 wt%, 3 wt%, 3.5 wt%, 4 wt% and 5 wt%.

The solid forest industry residue is typically dried to a moisture content below 20 wt%, preferably below 15 wt%, more preferably between 10 wt% and 20 wt% and most preferably between 10 wt% and 15 wt% before it is entered into the process of the disclosure.

## Fractionation of the product mix

The obtained product mix is fractionated in a fractionation step before being used as a renewable product and recirculated as recycled product fraction. A gaseous fraction, if any, one or more light and/or middle fraction(s) and a heavy fraction as well as a bottom residue fraction comprising solids, if any, may be obtained. The fractionation may be carried out after washing, filtering and/or drying the product mix. At least part of the middle fraction(s) is recirculated, i.e. used as the sole solvent in the feed mixture.

The gaseous fraction typically comprises water, carbon dioxide and light gaseous ($C_1$-$C_4$) compounds.

The vacuum residue of the product mix is typically above 10 wt.%. The heavy fraction may be directed to hydrocracking, energy production, recovery boiler, etc.

In an embodiment, part of the liquid product mix may be directed to a hydroprocessing step without fractionation.

In embodiments of the disclosure, one of the fractions, typically a light or middle fraction, obtained by fractionation of said product mix or liquid product mix, may be directed to a hydroprocessing step and at least part of the fraction(s) is used as solvent in the feed mixture and/or in the pre-treatment step of the process.

The fractionation may be carried out as evaporation, distillation, extraction, ultra-filtration, nanofiltration or as a combination of any of these.

Fractions comprising the gaseous fraction, the light and/or middle liquid fraction(s) and the heavy liquid fraction can be separated. A person skilled in the art is able to vary the fractionation/distilling conditions and to change the temperature cut point as desired to obtain any desired fraction, boiling in the predetermined ranges and comprising compounds which affects the pressure during the thermal liquefaction when the recycled product fraction is used as solvent in the feed mixture.

Alternatively, a combination of different methods may also be used.

## Optional hydroprocessing step

In the embodiments of the disclosure, the product mix, the light fraction obtained for example by fractionation of the product mix, or any other fraction of the product mix, may be subjected to a catalytic hydroprocessing step carried out in the presence of hydrogen, to yield an effluent, which may be subjected to a second fractionation and/or further processing steps for providing liquid fuels and other chemicals. Gasoline fractions that can be used as a bio-naphtha component or as raw material for bioplastics may also be produced.

The hydroprocessing step may be carried out for effecting at least one of hydrodeoxygenation, hydrodewaxing, hydroisomerization, hydrocracking, hydrodearomatization and ring opening reactions.

Hydroprocessing may be performed using one or more hydroprocessing catalysts comprising one or more metals selected from Group VIA and Group VIII metals (Periodic Table of Elements). Particularly useful examples are Mo, W, Co, Ni, Pt and Pd. The catalyst(s) can also contain one or more support materials, for example zeolite, alumina (Al2O3), gamma-alumina, zeolite-alumina, alumina-silica (SiO2), ZrO2, alumina-silica-zeolite and activated carbon. Suitably a mixture of CoO and MoO3 (CoMo) and/or a mixture of NiO and MoO3 (NiMo), and/or a mixture of Ni, Mo and Co and/or NiW and one or more support materials selected from zeolite, alumina, silica, zeolite-alumina, alumina-silica, alumina-silica-zeolite and activated carbon. Also, noble metals, such as Pt and/or Pd dispersed on gamma-alumina may be used.

In an embodiment, the hydroprocessing is carried out under a pressure of 5 - 300 bar (total pressure, abs). In an embodiment, the pressure in the hydroprocessing is from 30 to 250 bar, suitably from 30 to 120 bar.

In an embodiment, hydrogen partial pressure is maintained in the range from 50 to 250 bar, suitably from 80 to 200 bar, particularly suitably from 80 to 110 bar.

The hydroprocessing is carried out at a temperature in the range of 100 to 450°C, suitably 280°C to 450°C, more suitably from 350°C to 400°C.

The hydroprocessing feed rate WHSV (weight hourly spatial velocity) of the feedstock oil is proportional to an amount of the catalyst. The WHSV of the feed material varies between 0.1 and 10, it is suitably in the range of 0.1- 5 and preferably in the range of 0.3 - 0.7.

The ratio of H2/feed varies between 600 and 4000 Nl/l, suitably of 1300-2200 Nl/l.

The feed is pumped to the hydroprocessing reactor at a desired speed. Suitably the feed rate LHSV (liquid hourly space velocity) of the feed material is in the range of 0.01-10 h-1, suitably 0.1- 5 h-1.

The hydroprocessing step may be carried out as at least one-step process or as at least two-step process.

The liquid hydrocarbon stream obtained from the hydroprocessing includes fuel grade hydrocarbons having a boiling point of at most 380°C according to ISO EN 3405. The person skilled in the art is able to vary the distilling conditions and to change the temperature cut point as desired to obtain any suitable hydrocarbon product, boiling suitably in the transportation fuel ranges.

EXAMPLES

**Analysis methods used in the examples**

Gas samples were analyzed with an off-line Varian rapid gas chromatograph RGA-450GC with two analytical columns (10 m Molsieve 5A and 10 m PPQ), and with an off-line Agilent 7890A gas chromatograph equipped with three analytical columns and three different detectors (0.9 m Supelco 12255 U column and TCD detector for the quantification of CO2 and H2S, 10 m DB1 column and FID detector for the quantification of light hydrocarbons and, 10 m Moisieve 5A column and TCD detector for the quantification of O2, H2, N2 and CO), using helium as carrier gas in all cases.

The molecular weight distribution of the lignin fractions was determined with a Gel Permeation Chromatograph (GPC) using a Dionex Ultimate 3000 Autosampler (column compartment and pump), Dionex Ultimate 3000 Diode Array Detector and as a reflective index detector, Shodex RI-101. The columns were PSS MCX columns, precolumn and two analytical columns of sulfonated divinylbenzen copolymer matrix (1000 Å and 100 000 Å). The syringe filters were 0.45 μm and glass sample bottles for STD samples. Sample filtration was performed with a Mini-Uniprep syringeless filter device PTFE or Nylon, 0.45 μm. For prefiltration 5 μm syringe filter was used.

The eluent was prepared from high quality deionized water of low resistivity (18 MΩ-cm or better) that contains as little dissolved carbon dioxide as possible was used to prepare the eluent. The water was also free of biological contamination (e.g., bacteria and molds) and particulate matter. The water was first degassed and after this, the water was poured carefully into 2000 ml measurement bottle and exactly 10.4 ml of 50 % NaOH were added. The standard samples for calibration were prepared by weighting approximately 50 mg of each standard into a 10 ml measuring bottle and ultrapure water was added and filled into a mark. Standards were filtered with PTFE 0,45 μm syringe filters. After running the calibration samples, calibration results were integrated and processed in the processing method and saved. The strong alkaline liquor samples were diluted normally 1:100 and filtered with PTFE syringe filters (0,45 μm) to vials. Ready samples were loaded into autosampler, and sample information was added to sample sequence. Injection volume was 20 μl. After the samples 1 M NaOH was injected as a sample to clean the column. The instrument parameters were the following flow rate 0.5 ml/min, eluent 0.1 M NaOH, column oven temperature 30 °C, isocratic run and run time 48 minutes. The results were integrated, and the weight average molecular weight Mw was reported. The results are reported as integers.

The molecular weight distribution of different product oil fractions produced in the process were determined with a Gel Permeation Chromatograph (GPC) of the Agilent 1200 series, applying IR and UV light (wavelength 254 nm) and 3 GPC PLgel 3 lm MIXED-E columns placed in series. The columns were operated at 40 °C and tetrahydrofuran (THF) was used as a solvent. Calibration was performed with a solution of polystyrene with molecular weights ranging from 162 to 30230 g/mol.

Calculation of average molecular weight

$$M_w = \frac{\sum w_i M_i}{\sum w_i} \qquad\qquad (Eq.2)$$

where: $M_i$ is the molecular weight of molecules $i$, $n_i$ is the number of molecules with molecular weight $i$ and $w_i$ is the

mass of the molecules with molecular weight *i. M$_w$* is weight average molecular weight.

The elemental composition of the liquid and solids was determined with an Interscience Flash 2000 elemental analyser. The water content of the aqueous phase was determined by Karl Fischer titrations using Hydranal composite 5, Metrohm 787 KFTitrino as titrant.

The contents of ash, volatile matter, moisture and fixed carbon in the lignin (proximate analysis) were determined by measuring weight loss upon heating. These constituents will add up to 100%. Ash content determination was performed by heating a lignin sample in air at a slow heating rate (5°C/min). Once the temperature reached 550 °C it was kept constant for 6 hours before the sample was weighted. The remaining weights measured at 520 °C represent the ash contents at these temperatures.

The combined content of fixed carbon and volatiles was determined by slowly heating a lignin sample (5 °C/min) in nitrogen to 950 °C where it was maintained for 10 minutes before it was weighted. The measured weight loss represents the combined content of water and volatiles. The remaining weight represents the content of fixed carbon.

The moisture content of the lignin was determined by a PMB-53 moisture analyzer of Adam Equipment.

**Calculations of mass balance and yields**

The mass balance distinguishes four different product phases - oil (o), aqueous phase (aq), gas (g) and solids (s). The produced amounts of each phase are determined as follows:

1. Oil - is the liquid phase collected after the experiment.
2. Aqueous phase organics (water soluble organics - WSO) remain in the oil phase. Only when 20 wt% of water was used in the slurry mixture of lignin and lights, a separate water layer was observed which contained almost no organics molecules.
3. Gas - From the known volume of produced gas and GC composition, the weight of total gas is calculated and the amount of measured N2 is subtracted. The known volume of produced gas and the average molar weight of 33 g/mol are used calculate the amount of gas produced. Nitrogen is subtracted based on the initial pressure and the approximate initial volume taken up by gas phase in the reactor at the start of an experiment.
4. Solids (char) - The amount of solids is determined directly by weighing dried solids when withdrawn from the oven.

Since all yields are given on dry lignin basis, the amount of dry lignin fed in the reactor is corrected for initial lignin moisture as follows:

$$m_{lignin,dry} = m_{lignin} \cdot (1 - w_{moisture,lignin}) \tag{Eq.4}$$

The gas and solid yields are calculated by:

$$Y_{product} = \frac{m_{product}}{m_{lignin,dry}} \tag{Eq.5}$$

$Y_S$ is used for the solids and $Y_G$ for the gas. The oil yield is calculated as 100 minus gas and char. When the yields are expressed on the lignin intake, all solids and gas are first ascribed to the lignin after that, the amount of gas produced from the lights only are subtracted from the gas production from the lignin. The oil yield from lignin follows from 100 minus gas and solids from lignin.

**Estimation of composition of product fraction**

Molecular weight is taken directly from the GPC output file. The areas are calculated using numerical integration (trapezoidal method).

$$Area = \int_{logMw,1}^{logMw,2} RID \ d(log M_W) \tag{Eq.7}$$

**Oxygen content**

With the applied recovery procedure, a small fraction of water always remained in the oil phase. Therefore, the oxygen content is corrected for the oxygen in water. The water content of the oil sample ($KFT_{oil}$ in wt.%) is known from Karl Fischer titration and therefore the obtained oxygen content including that of water ($O_{wet}$) can be corrected to obtain the oxygen content of the oil on dry basis (O):

$$O = \frac{O_{wet} - \frac{16}{18} \cdot KFT_{oil}}{\left(1 - \frac{KFT_{oil}}{100}\right)} \qquad (Eq.8)$$

The same procedure holds for the hydrogen content in the oil:

$$H = \frac{H_{wet} - \frac{2}{18} \cdot KFT_{oil}}{\left(1 - \frac{KFT_{oil}}{100}\right)} \qquad (Eq.9)$$

## Example 1. Start-up of the process

A lignin feedstock with 35 wt% moisture was dried to a moisture content below 1 wt%. 3.2 kg or 6 kg of the dried lignin feedstock of Table 1 (8 or 15 wt.% by dry weight) and 36.8 or 34 kg of m-cresol, used as model component for a light fraction, respectively were mixed. The mixtures were placed in reactor vessels and heated to a temperature of 120 °C at 1 - 2 bar pressure. Cresol was selected as model compound for the light phase of the produced solvolysis oils and represents the recirculation in which the solvolysis of lignin occurs. The mix was mixed for 60 minutes, and the obtained feed mixture was used as pumpable feedstock for the continuous solvolysis plant.

Table 1. Kraft lignin composition of dried lignin feedstock

| Ash, wt% | < 1 |
|---|---|
| Organics, wt% | 99 |
| Moisture, wt% | < 1 |
| Molecular weight Mw, g/mol | 3406 |

## Example 2. Continuous thermal liquefaction step

The feed mixtures obtained in Example 1 were used for continuous solvolysis. The reactor temperature was 380 °C and the reaction time was 10 minutes (at set-point). The feeding rate was 5-10 kg/hr and the pressure was constant between 40 and 45 bar. The cooling temperature of the product vessel was 30 °C. The experiment with 8 wt% of dry lignin is shown in Table 2. The oil yield was 93 wt% and contains about 2 wt% of water. Similar results were obtained with 15 wt% of dry lignin. The lignin feedstock had an oxygen content of 30.1 wt%. The lignin/m-cresol slurry had an oxygen content of 16.1 wt%. The oxygen content of the slurry was reduced to 14 wt% after solvolysis. The de-oxygenation comes predominately from the lignin as the m-cresol is thermally stable.

Table 2. Yields after solvolysis of 8 wt% of dry lignin feedstock

| | Oil yield wt% | Solid yield wt%, dry | Gas yield wt%, dry | Oxygen wt%, dry | VR % |
|---|---|---|---|---|---|
| Feed, 8 wt% lignin, dry | 93 | 1 | 6 | 14 | 28 |

## Example 3. Fractionation of the solvolysis crude

The solvolysis crude, the oil yield obtained according to Example 2, was fractionated using vacuum distillation. The unit most frequently used in vacuum technology is millibar (mbar) and absolute vacuum is 0 mbar. The fraction obtained were the following ones

- A volatile fraction that boiled at a temperature below 120 °C at 1000 mbara. This fraction had a molecular weight distribution range from 18 to 100 g/mol.

- A light distillation fraction that had an average molecular weight of approximately 50 - 200 g/mol. The boiling point of the light distillation fraction was below 180 °C, at 20 mbar absolute (mbara) pressure. The distillation fraction comprised 5- and 6-carbon cyclic structures with double bonds or saturated ring, containing keto-groups and/or 1-3-methyl groups, phenolic structures with methyl-, methoxy-, propenyl-, carboxyl- side groups, and hydrated phenantrene 3-ring structures with 3-4 double bonds in the ring and side chains.
- A middle distillation fraction having a boiling point between 180 °C and 350 °C, at 20 mbar absolute (mbara) pressure. This distillation fraction comprised β-aryl ether substructures (β-O-4); B: resinol substructures (β-β); C: phenylcoumaran substructures (β-5), biphenyl substructures (5-5) and/or biphenyl ether (5-O-4). It had a molecular weight between 150 g/mol and 500 g/mol. This middle distillation fraction was solid at room conditions but become liquid at about 80 °C.
- A heavy distillation fraction obtained in the distillation process at temperatures higher than 350 °C. at 20 mbara. This heavy distillation fraction had an average molecular weight of above 450 g/mol and it is liquid at about 300 - 350 °C.

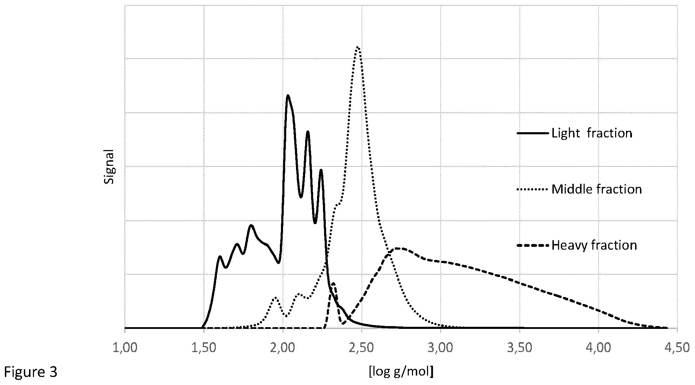

The molecular weight distribution for the different fractions obtained in the distillation of the solvolysis crude are shown in Figure 3.

The figure shows that some heavier compounds were present in the light distillation fraction and that lighter compounds were also present in the middle and heavy distillation fractions.

## Example 4. Solvolysis of lignin in the light fraction - Comparative Example

A mixture containing 15 wt% dry lignin, according to Table 1, in 85 wt-% of a light distillation fraction produced according to example 3 was fed to a reactor. The reactor temperature of the thermal liquefaction step was 380 °C and the reaction time was 10 minutes (at set-point). The yields on total organics as well as on lignin basis are shown in Table 3. The yield on lignin basis (between brackets [ ]) was corrected for the gas produced from the solvent (the light distillation fraction). The lignin produced only 5.4 wt% of gas and almost no solids.

Table 3. Yields on total organics and on lignin

| | Water content of feed mix (wt-%) | Gas yield (wt%, dry) | Solid yield (wt-%, dry) | Oil yield (wt%, dry) | Oxygen content (wt-%, dry) | Mw (g/mol) | Pressure (bar) |
|---|---|---|---|---|---|---|---|
| Lights fraction | - | 1.0 | - | 99.0 | 15 | 151 | 37 |
| Lights + dry lignin | 1 | 1.6 [5.4] | 0.2 [1.1] | 98.2 [93.6] | 16 | 423 | 65 |

The liquid product was fractionated, and the light fraction was used as solvent in the pre-treatment step. The light fraction can also be hydrotreated. Alternatively, or in addition the liquid product mix from the thermal liquefaction can as such, without fractionation, be hydrotreated and/or recirculated to the pre-treatment.

## Example 5. Stability of a middle fraction

The stability of the middle distillation fraction obtained according to example 3 was tested by heating the distillation fraction to the solvolysis temperature and changing the residence time. After the tests, the samples were analyzed. The results are shown in table 4.

Table 4. Middle distillation fraction at solvolysis temperature as a function of time.

| Solvent | T (°C) | Residence time (min) | Product distribution (wt-%) | | | Oxygen content (wt-%, dry) | M$_W$ (g/mol) | Water in solvent (wt-%) | P (bar) |
|---|---|---|---|---|---|---|---|---|---|
| | | | Solid | Gas | Liquid | | | | |
| Middle fraction feed | - | - | - | - | - | 18 | 309 | <1 | |
| Middle fraction | 370 | 30 | 0.0 | 1.2 | 98.8 | 14 | 496 | 3.6 | 23 |
| Middle fraction | 370 | 60 | 0.0 | 1.1 | 98.9 | 12 | 805 | 4.5 | 24 |
| Middle fraction | 370 | 120 | 2.0 | 1.2 | 96.8 | 11 | 848 | 8.6 | 39 |

Based on the increase in the molecular weight distribution, it is concluded that polymerization reactions took place that also contributed to the decrease on the oxygen content of the product. The oxygen was removed in the form of water, as seen in the water content after the tests. The water formed also had an effect on the pressure of the system.

**Example 6. Solvolysis of lignin in a middle fraction**

A mixture containing 15 wt% dry lignin, according to Table 1, in 85 wt-% of the middle distillation fraction produced according to Example 3 was fed to a reactor. The reactor temperature of the thermal liquefaction step was 370 °C and the residence time was 20 minutes (at set-point). The operation conditions, yields on total organics and product quality are shown in Table 5.

Table 5. Yields on total organics and on lignin

| | T (°C) | Residence time (min) | Product distribution (wt-%) | | | Oxygen (wt-%) | Mw (g/mol) | P (bar) |
|---|---|---|---|---|---|---|---|---|
| | | | Solid | Gas | Liquid | | | |
| Middle fraction only | 370 | 20 | 0.4 | 1.7 | 97.9 | 14.2 | 380 | 16 |
| Middle fraction + lignin | 370 | 20 | 0.4 | 1.7 | 97.9 | 13.2 | 663 | 27 |

As shown in Table 5, high liquid yields are reached. The reactions involving the liquefaction of lignin increased the pressure from 16 bar (for only the middle fraction at the reaction conditions) to 27 bar (middle fraction + lignin). The results in Table 3 and Table 5, demonstrate that utilizing middles as solvent in the thermal liquefaction of lignin allows for lower operation pressures and similar produced quality than when a lighter solvent is used.

The pressure in the solvolysis system is needed to keep the solvent in liquid form. Thus, using molecules having a higher boiling point (middle distillation fraction) gives the possibility to utilize lower pressures.

**Claims**

1.    A process for converting lignin to renewable liquid product(s), **characterized in that** the process comprises the following steps,

(a) providing a feed mixture (30) of lignin feedstock (10) or lignin-based feedstock (10), and recycled product fraction (20);
(b) treating said feed mixture (30) in a thermal liquefaction step by heating (110) the feed mixture (30) at a temperature between 300 and 420 °C, and maintaining said temperature for 2 - 60 minutes at a pressure below 35 bar; and

(c) obtaining a product mix (40),

wherein the recycled product fraction (20) is provided by separating (120) a liquid product mix (50) and an aqueous phase (53) from said product mix (40), fractionating (200) one or more fraction(s) (60, 61, 62) from said liquid product mix (50), or part thereof, at least a first fraction (61) having a boiling point between 300 °C and 600 °C at atmospheric pressure, and wherein at least part of said first fraction (61) is used as said recycled product fraction (20).

2.  The process according to claim 1, **characterized in that** said recycled product fraction further comprises a second fraction (60) having a boiling point between above 120 °C and below 300 °C at atmospheric pressure.

3.  The process according to any one of the preceding claims, **characterized in that** said recycled product fraction consist of 60 - 100 wt-% of said first fraction and 0-40 wt-% of said second fraction.

4.  The process according to any one of the preceding claims, **characterized in that** said first fraction essentially consists of di-, tri and tetra-aromatics.

5.  The process according to any one of the preceding claims, **characterized in that** said second fraction essentially consists of monoaromatics.

6.  The process according to any one of the preceding claims, **characterized in that** the pressure during the thermal liquefaction step is between 1 and 35 bar, more preferably between 1 and 30 bar, most preferably between 1 and 10 bar.

7.  The process according to any one of the preceding claims, **characterized in that** the process further comprises providing lignin feedstock by filtration, nanofiltration, acid precipitation process or membrane fractionation of a lignin source.

8.  The process according to any one of the preceding claims, **characterized in that** the lignin-based feedstock comprises 30 - 99 w-% lignin feedstock and 1 - 70 w-% other renewable feedstock, preferably solid forest industry residues, more preferably bark, sawdust, wood chips, branches, treetops, or mixtures thereof, most preferably sawdust.

9.  The process according to any one of the preceding claims, **characterized in that** the process further comprises preparing the feed mixture (30) by mixing (100) lignin feedstock (10) or lignin-based feedstock (10) with recycled product fraction (20), at a temperature between 80 and 220 °C, at a pressure from 1 bar to 10 bar, for 5 - 30 min.

10. The process according to claim 1 - 4, **characterized in that** the process further comprises preparing the feed mixture (30) by mixing (100) lignin feedstock (10) or lignin-based feedstock (10) with recycled product fraction (20), at a temperature between 80 and 220 °C, under at a pressure from 1 bar to 10 bar, for 5 - 30 min, followed by filtration and obtaining the feed mixture of lignin and solvent as the filtrate.

11. The process according to any one of the preceding claims, **characterized in that** the first fraction is a fraction corresponding to a fraction having a boiling point between 300 °C and 600 °C at atmospheric pressure, preferably a fraction having a boiling point between 180 °C and 350 °C at a 20 mbar absolute pressure.

12. The process according to any one of the preceding claims, **characterized in that** the second fraction is a fraction corresponding to a fraction having a boiling point between above 120 °C and below 300 °C at atmospheric pressure, preferably a fraction having a boiling point between above 100 °C and below 180 °C at a 20 mbar absolute pressure.

13. The process according to any one of the preceding claims, **characterized in that** the first fraction and/or second fraction does not comprise volatiles.

14. The process according to any one of the preceding claims, **characterized in that** the recycled product fraction comprising at least a part of the first fraction and optionally at least a part of the second fraction does essentially not consist of compounds having a boiling point below 120 °C at atmospheric pressure.

15. The process according to any one of the preceding claims, **characterized in that** the recycled product fraction comprising at least a part of the first fraction and optionally at least a part of the second fraction does essentially not comprise a fraction having a boiling point above 600 °C at atmospheric pressure.

16. The process according to any one of the preceding claims, **characterized in that** the lignin feedstock (10) has a molecular weight below 6500 g/mol, preferably below 4500 g/mol, more preferably below 3500 g/mol, most preferably below 2000 g/mol or below 1500 g/mol.

17. The process according to any one of the preceding claims, **characterized in that** the lignin feedstock (10) comprises 15-98 wt%, preferably 30 - 98 wt%, more preferably 30 - 70 wt%, most preferably 50 - 70 wt% organics, 0.1 - 15 wt%, preferably 0.1-7 wt%, more preferably 0.1 - 2 wt%, most preferably 0.1 - 1 wt% ash and/or 0 - 45 wt%, preferably 0 - 35 wt%, more preferably 0 - 20 wt%, most preferably 0 - 5 wt% moisture.

18. The process according to any one of the preceding claims, **characterized in that** the lignin feedstock (10) comprises below 40 wt%, preferably from 20 to 36 wt%, most preferably from 25 - 36 wt% oxygen daf (dry-ash-free).

19. The process according to any one of the preceding claims, **characterized in that** the process is a continuous, batch or semi-batch process.

20. The process according to any one of the preceding claims, **characterized in that** the lignin feedstock (10) is kraft lignin separated from black liquor and the lignin feedstock (10) comprises less than 10 wt% of, preferably less than 5 wt%, more preferably less than 1 wt%, most preferably less than 0.1 wt% of impurities or residues from the black liquor.

21. The process according to any one of the preceding claims, **characterized in that in that** at least part of the fraction(s) (60, 61, 62) from said fractionation (200) is directed to a hydroprocessing step.

22. The process according to any one of the preceding claims, **characterized in that** the amount of lignin feedstock or lignin-based feedstock is 5 wt% to 35 wt%, preferably the 8 wt% to 25 wt%, more preferably 10 to 20 wt% of the feed mixture and the rest is recycled product fraction.

23. The process according to any one of the preceding claims, **characterized in that** the thermal liquefaction of step (b) (110) is performed at a temperature from 300°C to 420°C, preferably at a temperature from 300 °C to 400 °C, more preferably at a temperature from 360 °C to 400 °C at a pressure from 1 to 35 bar, preferably from 1 to 30 bar, more preferably 1 to 10 bar and/or for 3 - 60 minutes, preferably 5 - 40 minutes, more preferably 5-30 minutes, most preferably 10 - 30 minutes.

24. Use of the product mix (40) or the liquid product mix (50) of any of claims 1 - 23 or any fractions thereof, as a renewable product, preferably in the production of chemicals, in marine fuel applications, in applications relating to heating or as feed in a hydroprocessing step.

25. Use of the hydroprocessed oil obtained by the process of claim 21 in the production of chemicals, marine fuel and/or drop-in fuels, preferably as diesel, naphtha or jet-fuel.

Figure 1

Figure 2

Figure 3

**EP 4 477 727 A1**

Europäisches
Patentamt

European
Patent Office

Office européen
des brevets

# EUROPEAN SEARCH REPORT

Application Number

EP 24 18 0447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/265794 A1 (DAHLSTRAND CHRISTIAN [SE] ET AL) 20 September 2018 (2018-09-20) * paragraphs [0022], [0032], [0042], [0049], [0051], [0055] - [0060], [0065], [0063], [0066], [0070], [0075], [0077] - [0082], [0096] - [0101] * | 1-25 | INV. C10G1/00 C10G1/02 C10G3/00 C07C37/54 C08L97/00 |
| X | US 4 900 873 A (KAKEMOTO GOHKI [JP] ET AL) 13 February 1990 (1990-02-13) * column 2, lines 24-38 * * column 3, lines 13-47 * * figure 1 * * examples 1, 2 * * comparative examples 1-4 * | 1-6, 11-15, 17-19, 21,22 | |
| X | WO 2021/209555 A1 (KVASIR TECH APS [DK]) 21 October 2021 (2021-10-21) * page 7, lines 6-33 * * page 8, lines 1-12 * * page 10, lines 10-17 * * page 11, lines 4-35 * * page 20, lines 4-16 * * page 18, lines 4-30 * | 1,3,4,6, 7,11,13, 17-19, 21-25 | TECHNICAL FIELDS SEARCHED (IPC) C10G C07C C08L |
| X A | EP 3 371 279 B1 (SCA FOREST PROD AB [SE]) 15 January 2020 (2020-01-15) * paragraphs [0044] - [0046] * | 24,25 1-23 | |
| X A | US 2013/060071 A1 (DELLEDONNE DANIELE [IT] ET AL) 7 March 2013 (2013-03-07) * paragraph [0132] * | 24,25 1-23 | |

The present search report has been drawn up for all claims

| Place of search | Date of completion of the search | Examiner |
|---|---|---|
| The Hague | 2 November 2024 | Baumlin, Sébastien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**

EP 24 18 0447

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2024

| Patent document cited in search report | Publication date | Patent family member(s) | Publication date |
|---|---|---|---|
| US 2018265794 A1 | 20-09-2018 | EP 3353269 A1 | 01-08-2018 |
| | | SE 1551213 A1 | 23-03-2017 |
| | | US 2018265794 A1 | 20-09-2018 |
| | | WO 2017052454 A1 | 30-03-2017 |
| US 4900873 A | 13-02-1990 | CA 1317601 C | 11-05-1993 |
| | | JP H0224258 B2 | 29-05-1990 |
| | | JP S6479127 A | 24-03-1989 |
| | | US 4900873 A | 13-02-1990 |
| WO 2021209555 A1 | 21-10-2021 | AU 2021255899 A1 | 27-10-2022 |
| | | BR 112022021041 A2 | 06-12-2022 |
| | | CA 3174184 A1 | 21-10-2021 |
| | | CN 115397950 A | 25-11-2022 |
| | | EP 4136189 A1 | 22-02-2023 |
| | | JP 2023521448 A | 24-05-2023 |
| | | KR 20220163489 A | 09-12-2022 |
| | | US 2023212463 A1 | 06-07-2023 |
| | | WO 2021209555 A1 | 21-10-2021 |
| EP 3371279 B1 | 15-01-2020 | CA 3004244 A1 | 11-05-2017 |
| | | DK 3371279 T3 | 03-02-2020 |
| | | EP 3371279 A1 | 12-09-2018 |
| | | ES 2773659 T3 | 14-07-2020 |
| | | LT 3371279 T | 25-02-2020 |
| | | PL 3371279 T3 | 18-05-2020 |
| | | PT 3371279 T | 19-02-2020 |
| | | WO 2017078582 A1 | 11-05-2017 |
| US 2013060071 A1 | 07-03-2013 | BR 112012023845 A2 | 02-08-2016 |
| | | EP 2550344 A2 | 30-01-2013 |
| | | IT 1399078 B1 | 05-04-2013 |
| | | US 2013060071 A1 | 07-03-2013 |
| | | WO 2011117705 A2 | 29-09-2011 |

EPO FORM P0459

For more details about this annex : see Official Journal of the European Patent Office, No. 12/82